(19) Europäisches Patentamt
European Patent Office
Office européen des brevets

(11) Veröffentlichungsnummer: **0 300 413 B1**

(12) **EUROPÄISCHE PATENTSCHRIFT**

(45) Veröffentlichungstag der Patentschrift: **01.09.93**

(51) Int. Cl.$^5$: **C07D 405/06**, A01N 43/653

(21) Anmeldenummer: **88111548.9**

(22) Anmeldetag: **18.07.88**

(54) **Mikrobizide Mittel.**

(30) Priorität: **20.07.87 CH 2746/87**

(43) Veröffentlichungstag der Anmeldung:
**25.01.89 Patentblatt 89/04**

(45) Bekanntmachung des Hinweises auf die Patenterteilung:
**01.09.93 Patentblatt 93/35**

(84) Benannte Vertragsstaaten:
**AT BE CH DE ES FR GB GR IT LI LU NL SE**

(56) Entgegenhaltungen:
**EP-A- 0 237 483**
**DE-A- 2 551 560**
**DE-A- 2 602 770**

(73) Patentinhaber: **CIBA-GEIGY AG**
**Klybeckstrasse 141**
**CH-4002 Basel(CH)**

(72) Erfinder: **Aebi, Rudolf**
**Holeestrasse 69**
**CH-4054 Basel(CH)**
Erfinder: **Hubele, Adolf, Dr.**
**Obere Egg 9**
**CH-4312 Magden(CH)**
Erfinder: **Speich, Jürg, Dr.**
**Unterer Rheinweg 46**
**CH-4057 Basel(CH)**

(74) Vertreter: **Zumstein, Fritz, Dr. et al**
**Bräuhausstrasse 4**
**D-80331 München (DE)**

**EP 0 300 413 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung des 2RS,4S-Diastereomerengemischs vom 1-[2-(2,4-Dichlorphenyl)-4-n-propyl-1,3-dioxolan-2-ylmethyl]-1H-1,2,4-triazol, sowie mikrobizide Mittel, die als Wirkstoff diese Verbindungen enthalten. Die Erfindung betrifft ferner das neue 2RS,4S-Diastereomerengemisch und die Verwendung der neuen Wirkstoffe bzw. der genannten Mittel zur Bekämpfung von schädlichen Mikroorganismen und zur Verhütung von Pilzbefall, insbesondere bei der Beizung von Saatgut.

Den isomeren Wirkstoffen liegt das unter dem Namen "Propiconazol" bekanntgewordene racemische Produkt 1-[2-(2,4-Dichlorphenyl)-4-n-propyl-1,3-dioxolan-2-ylmethyl]-1H-1,2,4-triazol der Formel

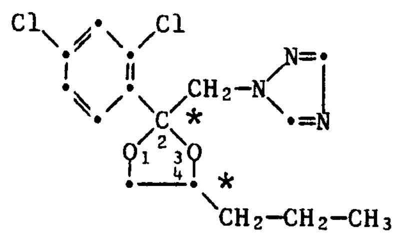

zugrunde, das aus der GB-A 1,522,657 als Fungizid bekanntgeworden ist. Es enthält an den markierten Stellen (*) zwei asymmetrische Kohlenstoffatome. Bei der Herstellung dieses "Propiconazols" bildet sich zu ca. 60 % das "cis-Racemat", zu ca. 40 % das "trans-Racemat". Das "cis-Racemat" enthält das Enantiomerenpaar mit der 2R,4S- bzw. 2S,4R-Konfiguration, das "trans-Racemat" das Enantiomerenpaar mit der 2S,4S- bzw. 2R,4R-Konfiguration. Auf diese Tatsache wird in der GB-A 1,522,657 nicht ausdrücklich hingewiesen.

Die vier reinen Enantiomeren weisen als Blattfungizide unterschiedliche Aktivitäten auf und lassen sich nach Wirkungsstärke etwa wie folgt einordnen: 2S,4R>2S,4S>2R,4S>2R,4R [E. Ebert et al: Structure Activity Relationships of Fungicidally Active Triazoles, (The Physicochemical and Biophysical Panel of the Society of Chem. Ind. 26.2.85, London)].

Es hat sich nun in den letzten Jahren vermehrt gezeigt, dass die zu den Ergosterinbiosynthese-Inhibitoren zählende Stoffklasse der 1H-1,2,4-Triazolylverbindungen in der Agrochemie nicht nur als Pflanzenfungizide, sondern auch als Pflanzenwuchshemmer einsetzbar ist. Unter der Fülle von Publikationen, in denen beide Eigenschaften als Anwendungsgebiet vorgeschlagen werden, seien als Beispiele EP-A-15 387, EP-A-32 200, EP-A-44 993, EP-A-53 311, EP-A-87 148, EP-A-123 160, EP-A-114 487, EP-A-47 594 genannt. In Nutzkulturen ist diese wuchshemmende oder wuchsregulierende Eigenschaft sehr häufig nicht erwünscht, da unerwartete Effekte im Pflanzenwuchs auftreten können. Bei der Anwendung an Obstbäumen zur Bekämpfung von Mehltau- und Rostbefall wird beispielsweise bei den reifenden Früchten sehr häufig Zwergwuchs beobachtet, der in der Praxis gleichbedeutend mit Ernteausfall ist. Im besten Falle besitzen 1H-1,2,4-Triazolyl-Fungizide nur eine geringe Phytotoxizität, wobei unter diesem Begriff jede Form der abweichenden Pflanzenentwicklung verstanden werden soll. Eine solche Nebenwirkung ist auch bei der Verwendung des 1-[2-(2,4-Dichlorphenyl)-4-n-propyl-1,3-dioxolan-2-ylmethyl]-1H-1,2,4-triazol als Blattfungizid und insbesondere bei der Saatbeizung zu beobachten. Als Saatbeizmittel hat Propiconazol keinen Eingang in die Praxis gefunden, weil auch bei vorsichtiger Anwendung die Keimfähigkeit von behandeltem Saatgut in der Regel deutlich vermindert ist und das Auflaufvermögen der Kultur zeitlich zu stark verzögert wird.

Aufgrund der jahrlangen intensiven Forschung hat sich in der Fachwelt die Meinung gebildet, dass 1H-1,2,4-Triazolderivate generell einen pflanzenwuchsbeeinflussenden, im allgemeinen pflanzenwuchshemmenden Effekt ausübten, der je nach Molekülstruktur stärker oder schwächer hervortrete, jedoch mindestens latent immer vorhanden sei.

Es hat sich nun ganz unerwartet gezeigt, dass das 2R,4S-Isomere des Propiconazols bei voller pflanzenmikrobizider Aktivität im Gegensatz zu den übrigen drei obengenannten Isomeren praktisch keine sichtbare Pflanzenwuchsbeeinflussung, also keine Phytotoxizität, aufweist und dass die Phytotoxizität des 2S,4S-Isomeren vergleichsweise gering ist.

Dies ist im Hinblick auf das vorher Gesagte überraschend, und es ist um so überraschender, als die GB-A-1,522,657 keine Hinweise auf unterschiedliche biologische bzw. mikrobizide oder phytotoxische Wirkung der Isomeren enthält.

Ziel der vorliegenden Erfindung ist die Bereitstellung einer nachhaltig mikrobiziden, besonders pflanzenfungiziden Aktivsubstanz ohne nachteilige phytotoxische Nebenwirkung, die insbesondere ihren Einsatz in Obstbaumkulturen und als Saatbeizmittel erlaubt.

Es wurde überraschend gefunden, dass das 2R,4S-Isomere der Formel I des 1-[2-(2,4-Dichlorphenyl)-4-n-propyl-1,3-dioxolan-2-ylmethyl]-1H-1,2,4-triazols diesen Anforderungen voll entspricht und dass auch das 2RS,4S-Diastereomerengemischder Formel Ia für die Mehrzahl der Fälle diesen Anforderungen genügt. Dieses 2RS,4S-Diatereomerengemisch setzt sich zu gleichen Teilen aus dem 2R,4S-Enantiomeren und dem 2S,4S-Enantiomeren zusammen und ist neu, denn weder das obenerwähnte "cis-Racemat" noch das "trans-Racemat" entsprechen dieser Isomerenzusammensetzung.

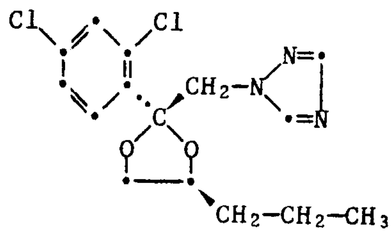

(I)
2R,4S

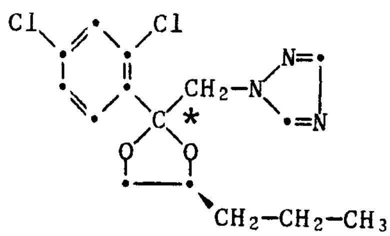

(Ia)
2RS,4S

Entscheidend für die Gewinnung ist, dass man von vornherein durch den gezielten Einsatz von S-1,2-Pentandiol der Formel II

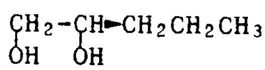

(II) S

zu Verbindungen mit 4S-Konfiguration gelangt. Damit wird die Entstehung von Verbindungen mit 4R-Konfiguration vermieden, die im Falle des Propiconazol im wesentlichen für die phytotoxischen Erscheinungen verantwortlich sind.

Das 2RS,4S-Diastereomerengemisch der Formel Ia wird erhalten, indem man das Diastereomerengemisch 2RS-4S-2-[2-(2,4-Dichlorphenyl)-2-brommethyl-4-n-propyl-1,3-dioxolan der Formel III mit 1,2,3,-Triazol der Formel IV

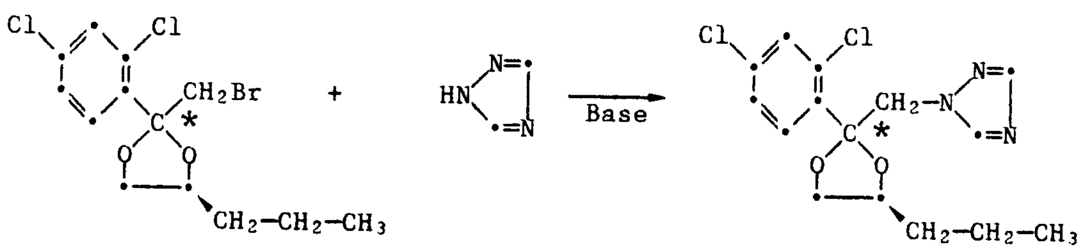


(III) 2RS,4S (IV) (Ia) 2RS,4S

in einem Lösungsmittel bei 10-160 ° C, in Gegenwart einer Base als Säureazkeptor zum Diastereomerengemisch 2RS,4S-1-[2-(2,4-Dichlorphenyl)-4-n-propyl-1,3-dioxolan-2-ylmethyl]-1H-1,2,4-triazol der Formel Ia umsetzt.

Aus dem 2RS,4S-Diastereomerengemisch kann das 2R,4S-1-[2-(2,4-Dichlorphenyl)-4-n-propyl-1,3-dioxolan-2-ylmethyl]-1H-1,2,4-triazol vom Diastereomeren 2S,4S-1-[2-(2,4-Dichlorphenyl)-4-n-propyl-1,3-dioxolan-2-ylmethyl]-1H-1,2,4-triazol abgetrennt und isoliert werden. Die Trennung kann nach einer der üblichen Methoden an einem Adsorption/Desorption-Material erfolgen, z.B. an einem sauren Ionenaustauscherharz oder an Kieselgel, beispielsweise durch Säulenchromatographie.

Das 2RS,4S-Diastereomerengemisch oder das reine 2R,4S-Isomere können gegebenenfalls durch Umsetzung mit einer salzbildenden Säure in ein Salz überführt werden. Beispiele solcher Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Dieses Verfahren, das zur Herstellung der Reinsubstanzen 2RS-4S- bzw. 2R,4S-1-[2-(2,4-Dichlorphenyl)-4-n-propyl-1,3-dioxolan-2-ylmethyl]-1H-1,2,4-triazol dient, ist neu und ist ebenfalls Gegenstand der vorliegenden Erfindung.

Als Lösungsmittel für die Umsetzung der Verbindung der Formel III mit 1,2,4-Triazol können - gegebenenfalls halogenierte - aliphatische oder aromatische Kohlenwasserstoffe, beispielsweise Toluol, Xylol, Chlorbenzol, o-Dichlorbenzol, Methylenchlorid, Tetrachlorkohlenstoff, Tetrachloräthylen, ferner aliphatische Aether, beispielsweise Diäthyläther oder Diisopropyläther, schliesslich apolare, aprotische Lösungsmittel, beispielsweise Dimethylformamid oder Dimethylsulfoxid eingesetzt werden.

Bevorzugt werden von den erwähnten Lösungsmittel die apolaren, aprotischen Lösungsmittel, insbesondere das Dimethylsulfoxid.

Bevorzugt werden als Basen die Alkalihydroxide, insbesondere das Kaliumhydroxid.

Der bevorzugte Temperaturbereich der Umsetzung liegt zwischen 130 und 150 ° C.

Als Lösungsmittel oder Laufmittel für die säulenchromatographische Trennung können Lösungsmittel mit geeigneter Polarität, wie gegebenenfalls halogenierte aliphatische oder aromatische Kohlenwasserstoffe, Ester, Aether oder deren Gemische verwendet werden. Bevorzugt werden als Lösungsmittel aliphatische Kohlenwasserstoffe und aliphatische Ester und der Gemische. Insbesondere wird das Aethylacetate/Hexan-Gemisch (1:1) bevorzugt. Die Trennung wird im Temperaturbereich von 10 ° -60 ° C durchgeführt, vorzugsweise bei Raumtemperatur bis ca. 40 ° C.

Die Trennung kann auch an anderen stationären Phasen, wie beispielsweise an Ionenaustauschern mit sauren Resten erfolgen.

Die Verbindung der Formel III kann durch Kondensation des 2',4'-Dichlor-2-bromacetophenons der Formel V mit S-1,2-Pentandiol der Formel II hergestellt werden.

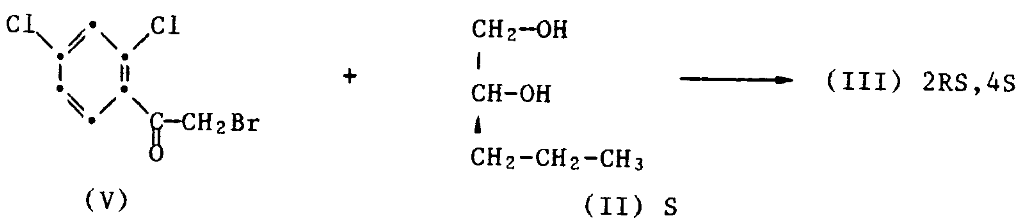
(V) + (II) S (III) 2RS,4S

Die Herstellung des Diols der Formel II erfolgt zweckmässig durch Reduktion der S-α-Hydroxyvaleriansäure der Formel VI mit einem geeigneten Reduktionsmittel, wie beispielsweise dem Boran-Dimethylsulfid-Komplex der Formel VII in Tetrahydrofuran:

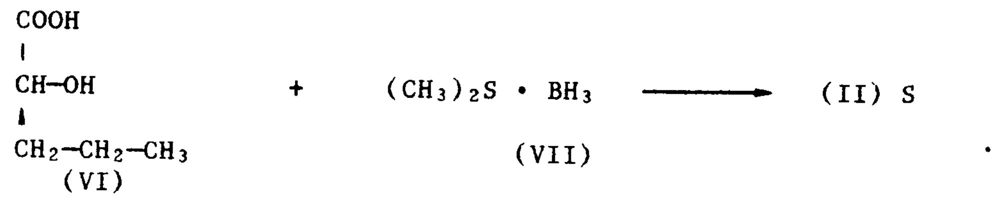

(VI) (VII) (II) S

Die Herstellung der Verbindung der Formel VI (S) ist im J. Am. Chem. Soc. 78, 2423 (1956) beschrieben. Ihre Reinigung kann über geeignete Salze, beispielsweise über das S(-)-1-Penyläthylammoniumsalz erfolgen.

Die Verbindungen der Formeln IV, V und VII sind im Handel erhältliche Chemikalien.

Die Verbindungen der Formel I und Ia sind insbesondere geeignet, Pflanzen vor Pilz- und Bakterienbefall dauerhaft zu schützen und ihre Entwicklung zu fördern. Sie stellen als pflanzenverträgliche mikrobizide Aktivsubstanzen eine ganz wesentliche Bereicherung der Technik dar.

Das Haupteinsatzgebiet der Verbindung der Formel I und Ia liegt in der Bekämpfung von schädlichen phytopathogenen Pilzen. So besitzen die Verbindungen der Formel I bzw. Ia eine für praktische Bedürfnisse sehr günstige kurative, präventive und systemische Wirkung zum Schutz von Kulturpflanzen, ohne diese durch unerwünschte Nebenwirkungen zu beeinflussen. Kulturpflanzen seien im Rahmen vorliegender Erfindung beispielsweise Getreide (Weizen, Gerste, Roggen, Hafer, Reis); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüseorten (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen, Avocados und Naturkautschukgewächse sowie Zierpflanzen.

Mit dem Wirkstoff der Formel I bzw. Ia können an Pflanzen oder an Pflanzenteilen (Früchten, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind vor allem gegen die den folgenden Klassen angehörenden phytopathogenen Pilze wirksam: Ascomycetes (wie Erysiphe, Venturia, Pyrenophora (= reife Form von Helminthosporium), Calonectria graminicola (= reife Form von Fusarium); Basidiomycetes wie Tilletia und Ustilago; Fungi imperfecti wie Helminthosporium, Fusarium, Septoria, Cercospora.

Die Verbindungen der Formel I und Ia können deshalb besonders als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Insbesondere werden Krankheitserreger bekämpft, die Getreidearten in irgendeinem Entwicklungsstadium befallen, sei es beim Auflaufen, bei der Bestockung, bei der Reifung, bei der Lagerung des Saatguts (Saatbeizung) oder bei der Aussaat. Als Getreidearten seien beispielsweise Weizen, Roggen, Gerste, Hafer, Reis, Mais und Kulturhirse genannt. Bekämpft werden durch die Verbindung Ia und besonders die Verbindung I unter anderen folgende bedeutende Krankheitserreger
Helminthosporium gramineum (Gerste, Weizen)
Helminthosporium oryzae (Reis)
Tilletia caries (Gerste, Weizen, Roggen)
Drechslera teres (Gerste, Weizen)
Ustilago tritici (Weizen)
Ustilago maydis (Mais)
Puccinia graminis (Weizen, Gerste, Roggen, Hafer)
Erysiphe graminis (Weizen, Gerste, Roggen, Hafer)
Fusarium nivale (Roggen)
Fusarium culmorum (Weizen)
Pseudocercosporella herpotrichoides (Roggen).

In Klammern sind nur die wichtigsten Kulturen aufgeführt.

Die Erfindung betrifft somit auch die Verwendung der Verbindungen der Formel I und Ia zur Bekämpfung phytopathogener Mikroorganismen bzw. zur präventiven Verhütung eines Befalls an Pflanzen wie Obstbäumen, die üblicherweise durch Triazol-Fungizide im Wuchs oder Fruchtansatz gehemmt oder anderweitig ungünstig beeinflusst werden.

Die Erfindung betrifft insbesondere die Verwendung der Verbindung Ia und vor allem der Verbindung I zur Beizung von Getreide-Saatgut, das üblicherweise durch Triazol-Fungizide eine Auflauf-Verzögerung erfährt oder generell ungenügend keimt. Durch die Bereitstellung des völlig pflanzenverträglichen 2R,4S-1-[2-(2,4-Dichlorphenyl)-4-n-propyl-1,3-dioxolan-2-ylmethyl]-1H-1,2,4-triazols wird das Problem des dauerhaften Schutzes von Getreide-Saatgut vor pilzlichen Krankheitserregern gelöst. Die Erfindung bezieht sich auch auf so behandeltes Saatgut.

Der jeweilige Wirkstoff der Formel I bzw. Ia wird üblicherweise in Form von Zusammensetzungen verwendet und kann gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungsm-, Dispergier-, Netz-, Haft-, Verdickungs-, Binde- oder Düngemitteln.

Die Verbindungen der Formel I bzw. Ia werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren werden gleich wie die Art der Mittel den angestrebten Zielen und den gegebenen Verhältnissen entsprechend gewählt. Im Agrarsektor liegen günstige Aufwandmengen im allgemeinen bei 50 g bis 5 kg Aktivsubstanz (AS) je hat; bevorzugt 100 g bis 2 kg AS/ha, insbesondere bei 100 g bis 600 g AS/ha.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin oder Lysolecithin.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual" BC Publishing Corp., Ringwood New Jersey, 1981;
Helmut Stache "Tensid-Taschenbuch" Carl Hanser-Verlag München/Wien 1981.
M. and J. Ash. "Encyclopedia of Surfactants", Vol. I-III, Chemical Publishing Co., New York, 1980-1981.

Die hierin beschriebenen pflanzenmikrobiziden Mittel enthalten in der Regel 0,01 bis 95 %, bevorzugt 0,1 % bis 60 % Wirkstoff auf Propiconazol-Basis, und zusätzlich einen oder mehrere übliche Trägerstoffe und/oder Netzmittel ( = oberflächenaktive Präparate).

Die Mittel sind gemäss vorliegender Erfindung dadurch gekennzeichnet, dass der Wirkstoff Propiconazol einen möglichst hohen, mindestens aber 45 %-igen ( = Gew.-%) Anteil an 2R,4S-Isomeren und kaum Anteile oder bevorzugt keine Anteile an 4R-Isomeren enthält. Je höher der Anteil des 2R,4S-Isomeren in der Propiconazol-Wirkstoffkomponente ist, desto problemloser lassen sich derartige Mittel zur Pflanzenapplikation, z.B. an Apfel- und Birnbäumen, besonders aber zur Saatbeizung, auch bei zufälliger Höherdosierung anwenden. Bevorzugt sind daher Mittel, bei denen der Propiconazol-Anteil zu mindestens 70 Gewichtsprozent, besonders zu mindestens 90 Gewichtsprozent oder zu 98-100 Gewichtsprozent aus dem pflanzenverträglichen 2R,4S-Isomeren der Formel I besteht.

Sofern das Diastereomerengemisch 2RS,4S-1-[2,(2,4-Dichlorphenyl)-4-n-propyl-1,3-dioxolan-2-ylmethyl]-1H-1,2,4-triazol eingesetzt wird (mit gleichen Anteilen an 2R,4S-Isomeren und 2S,4S-Isomerem), beträgt sein Anteil am Propiconazol-Gesamt-Wirkstoff demgemäss mindestens 90 Gew.-%. Ein solcher Anteil, der noch 10 Gew.-% oder weniger phytotoxisches 4R-Isomeres enthalten kann, gewährleistet in der

Mehrzahl der Praxisfälle eine noch hinreichende Pflanzenverträglichkeit. Den erfindungsgemässen Mitteln kann also in geringen Mengen beispielsweise racemisches Propiconazol beigemischt sein, so dass der Anteil an 4R-Isomeren den Betrag von 10 Gew.-% nicht überschreitet, bevorzugt jedoch unter 5 Gew.-% liegt und besonders bevorzugt Null ist.

Während als Handelsware eher konzentrierte Mittel bevorzugt werden, verwendet der Endverbraucher in der Regel vedünnte Mittel mit Konzentrationen von 0,001 % bis 1 %.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen der Illustration der Erfindung, ohne dieselbe einzuschränken.

## 1. Herstellungsbeispiele

### Beispiel 1: Reinigung der S-α-Hydroxyvaleriansäure

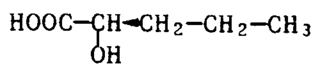

Die nach M. Winitz et al [J. Amer. Chem. Soc. 78, 2423 (1956)] hergestellte rohe Säure wird durch Kristallisation ihres S(-)-1-Phenyläthylammoniumsalzes aus Acetonitril gereinigt. Das reine Salz schmilzt bei 96-99 °C; $[\alpha]_D^{20}$ = -20 ° ± 1 ° (c = 3,164 in Methanol).

90 g (0,4 Mol) des gereinigten Salzes werden in 1,5 Liter Dioxan suspendiert und mit 40 ml konz. Salzsäure versetzt. Das Lösungsmittel wird weitgehend verdampft, der viskose Rückstand erneut in 500 ml Dioxan gelöst, das S-1-Phenyläthylaminhydrochlorid mit 2,5 Liter Diäthyläther aufgefällt, der Niederschlag filtriert, mit Diäthyläther nachgewaschen und das Lösungsmittel aus dem Filtrat im Vakuum verdampft. Es werden 61 g der S-α-Hydroxyvaleriansäure in Form eines hellgelben Oeles erhalten, das noch ca. 20% Dioxan enthält.

### Beispiel 2: Herstellung des S-1,2-Pentandiols

61 g (~0,42 Mol) der S-α-Hydroxyvaleriansäure, die noch ca. 20% Dioxan enthält, werden in 600 ml Tetrahydrofuran gelöst. Zu dieser Lösung werden innerhalb einer Stunde unter Rühren bei 55-65 °C 64 ml (0,64 Mol) Boran-Dimethylsulfid-Komplex zugetropft, vier Stunden unter Rückfluss erhitzt, auf Raumtemperatur gekühlt und in der Kälte mit 160 ml Methanol versetzt. Nach beendeter Wasserstoffentwicklung werden 80 ml 0,1 N Salzsäure zugefügt, 1/2 Stunde gerührt und das Lösungsmittel im Vakuum verdampft. Der teils kristalline Rückstand wird mit 2 Liter Däthyläther verrührt, die ausgefallene Borsäure abfiltriert und das Filtrat eingedampft. Der Rückstand wird in 650 ml Wasser gelöst, mit Bariumhydroxyd auf pH 13 gebracht, über Kieselgel filtriert und das Filtrat bei 60 °C im Vakuum auf ca. 300 ml eingedampft. Durch Zugabe von 800 ml Aceton wird das Bariumborat ausgefällt, abfiltriert, das Nutschgut mit Aceton nachgewaschen und die vereinigten Filtrate eingedampft. Der Rückstand wird in 1,5 Liter Diäthyläther aufgenommen, über Natriumsulfat getrocknet, filtriert und das Lösungsmittel verdampft. Nach Destillation des Rückstands erhält man 33 g (76 % der Theorie) S-1,2-Pentandiol als farbloses Oel, Kp. 106-108/21 mbar, $[\alpha]_D^{20}$ = -15 ° ± 1 ° (c = 3,028 % in Methanol).

### Beispiel 3: Herstellung des 2RS,4S-2-(2,4-Dichlorphenyl)-2-brommethyl-4-n-propyl-1,3-dioxolans

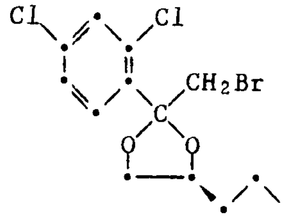

77,7 g (0,29 Mol) 2',4'-Dichlor-2-bromacetophenon, 33 g (0,32 Mol) S-1,2-Pentandiol und 2,5 g p-Toluolsulfonsäure in 500 ml Toluol werden 16 Stunden am Wasserabscheider unter Rückfluss erhitzt. Nach

dem Abkühlen auf Raumtemperatur wird mit 750 ml Diäthyläther verdünnt, die Lösung zur Beseitigung der p-Toluolsulfonsäure mit 200 ml I N Sodalösung behandelt, mit Wasser neutral gewaschen, über Natriumsulfat getrocknet und das Lösungsmittel verdampft. Nach Destillation des Rückstands werden 72,5 g (70,6 % der Theorie) 2RS,4S-2-(2,4-Dichlorphenyl)-2-brommethyl-4-n-prop-yl-1,3-dioxolan erhalten; Kp. 125-129°/$10^{-3}$ mbar.

Beispiel 4: Herstellung des Diastereomerengemisches 2RS,4S-1-[2-(2,4-Dichlorphenyl)-4-n-propyl-1,3-dioxolan-2-ylmethyl]-1H-1,2,4-triazol

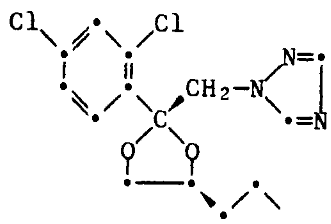

41,4 g (0,6 Mol) 1,2,4-Triazol werden in 350 ml Dimethylsulfoxid mit 33 g (0,5 Mol) 85%igem KOH versetzt und bei 45°C bis zur Bildung einer klaren, farblosen Lösung gerührt. Zu dieser Lösung werden 126 g (0,356 Mol) des Diastereomerengemisches 2RS,4S-2-(2,4-Dichlorphenyl)-2-brommethyl-4-n-propyl-1,3-dioxolan in 50 ml Dimethylsulfoxid zugegeben und das Gemisch 17 Stunden bei 140°C gerührt. Nach dem Abkühlen auf Raumtemperatur wird das Reaktionsgut mit 1,5 Liter Wasser verrührt, mit 2 Liter Diethylether extrahiert, die Etherphase neutral gewaschen, über Natriumsulfat getrocknet, filtriert und das Lösungsmittel verdampft. Nach der Destillation des Rückstands werden 89,3 g (73 % der Theorie) des Diastereomerengemisches 2RS,4S-1-[2-(2,4-Dichlorphenyl)-4-n-propyl-1,3-dioxolan-2-ylmethyl]-1H-1,2,4-triazol erhalten; Sdp. 155-160°/$10^{-3}$ mbar.

Beispiel 5: Herstellung des Isomeren 2R,4S-1-[2,4-Dichlorphenyl)-4-n-propyl-1,3-dioxolan-2-ylmethyl]-1H-1,2,4-triazols

Die Auftrennung des destillierten Produkts in die beiden Isomeren erfolgt durch Säulenchromatographie über Kieselgel mit Essigsäureäthylester/Hexan (1:1) als Laufmittel. Es werden 38,3 g 2R,4S-1-[2-(2,4-Dichlorphenyl)-4-n-propyl-1,3-dioxolan-2-ylmethyl]-1H-1,2,4-triazol: $[\alpha]_D^{20}$ = +13° ± 1° (c = 4,142 % in Methanol) und als Nebenprodukt 28,4 g 2S,4S-1-[2-(2,4-Dichlorphenyl)-4-n-propyl-1,3-dioxolan-2-ylmethyl]-1H-1,2,4-triazol, Oel: $[\alpha]_D^{20}$ = -5° ± 1° (c = 3,17 % in Methanol) erhalten.

2. Formulierungsbeispiele für die Wirkstoffe der Formel I und Ia

[In Klammern Prozentsatz an 2R,4S-Isomeren im Propiconazol-Wirkstoff]

| 2.1. Emulsion-Konzentrate | | | |
|---|---|---|---|
| | a) | b) | c) |
| Wirkstoff [70 % 2R,4S] | 25% | 40% | 50% |
| Ca-Dodecylbenzolsulfonat | 5% | 8% | 6% |
| Ricinusöl-polyäthylenglykoläther (36 Mol Aethylenoxid | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden, die sich für die Blattapplikation oder die Nassbeizung eignen.

| 2.2. Lösungen | | | | |
|---|---|---|---|---|
| | a) | b) | c) | d) |
| Wirkstoff [90 % 2R,4S] | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190 °C) | - | - | 94% | - |
| (MG = Molekulargewicht) | | | | |

| 2.3. Granulate | | |
|---|---|---|
| | a) | b) |
| Wirkstoff [45 % 2R,4S] | 5% | 10% |
| Kaolin | 94% | |
| Hochdisperse Kieselsäure | 1% | - |
| Attapulgit | - | 90% |

Der Wirkstoff wird in Methylenchlorid gelöst, auf den Träger ausgesprüht und das Lösungsmittel anschliessend im Vakuum abgedampft.

| 2.4. Stäubemittel | | |
|---|---|---|
| | a) | b) |
| Wirkstoff [98 % 2R,4S] | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel. Solche Stäubemittel sind zur Trockenbeizung von Getreide-Saatgut geeignet.

| 2.5. Spritzpulver | |
|---|---|
| Wirkstoff [80 % 2R, 4S] | 25% |
| Na-Ligninsulfonat | 5% |
| Na-Laurylsulfat | 3% |
| Na-Diisobutylnaphthalinsulfonat | - |
| Octylphenolpolyäthylenglykoläther (7-8 Mol Aethylenoxid | - |
| Hochdisperse Kieselsäure | 5% |
| Kaolin | 62% |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen. Solche Suspensionen sind für die Beizung von Getreidesaat geeignet.

| 2.6. Emulsions-Konzentrat | |
|---|---|
| Wirkstoff [70 % 2R,4S] | 10% |
| Octylphenolpolyäthylenglykoläther (4-5 Mol Aethylenoxid) | 3% |
| Ca-Dodecylbenzosulfonat | 3% |
| Ricinusölpolyglykoläther (35 Mol Aethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7. Extruder Granulat | |
|---|---|
| Wirkstoff [50 % 2R,4S] | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wurde extrudiert und anschliessend im Luftstrom getrocknet.

| 2.8. Umhüllungs-Granulat | |
|---|---|
| Wirkstoff [45 % 2R,4S] | 3% |
| Polyäthylenglykol M G 200 | 3% |
| Kaolin | 94% |
| (MG = Molekulargewicht) | |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate. Dosiert man im gleichen Mischer nach einiger Zeit Getreide-Saatgut dazu, erhält man im selben Arbeitsgang beschichtetes, bzw. umhülltes Saatgut.

| 2.9. Suspensions-Konzentrat | |
|---|---|
| Wirkstoff [93 % 2R,4S] | 40% |
| Aethylenglykol | 10% |
| Nonylphenolpolyäthyleglykoläther (15 Mol Aethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können. Solche Suspensionen sind für die Beizung von Getreidesaat (Tauchbeizung, Sprühbeizung) geeignet.

<u>Benetzungsbeize</u>

80 g trockene Getreidekörner (z.B. Mais) werden in veschliessbaren Plastikbechern mit 2R,4S-Propiconazol der Formel I oder mit 2RS,4S-Propiconazol der Formel Ia in Form einer wässrigen Suspension, Emulsion oder Lösung gut durchmischt.

Die Substanzapplikation wird so bemessen, dass eine Wirkstoff-Konzentration von 0,06 bis 0,001 %, bezogen auf das Korntrockengewicht, erreicht wird.

## 3. Biologische Beispiele

### Beispiel 3.1: Saatbeize von Weizen mit Stereoisomeren des 1-[2-(2,4-Dichlorphenyl)-4-n-propyl-1,3-dioxolan-2-ylmethyl]-1H-1,2,4-triazols und mit dessen handelsüblichem Gemisch[1], gegen Erysiphe graminis

Mit Erysiphe graminis auf künstliche Weise infiziertes Weizensaatgut wird mit den zu prüfenden Fungiziden in einer Konzentration von jeweils 6 g Aktivsubstanz/100 kg Saatgut gebeizt (= 60 ppm AS).

Für jedes zu prüfende Fungizid werden 3 x 100 Weizenkörner in 3 Saatschalen, gefüllt mit Naturboden, gesetzt und im Treibhaus unter denselben Bedingungen gehalten.

Die Aktivität der Fungizide wurde 30 Tage nach der Aussaat aufgrund der durchschnittlich vom Pilz befallenen prozentualen Fläche berechnet, und die Auflaufverzögerung durch Messung der Wuchshöhe nach 14 und nach 19 Tagen. Zum Vergleich lief ein Versuch mit unbehandeltem, infizierten Weizen mit.

Dieser unbehandelte Weizen wies nach 14 und 19 Tagen 2,2 cm bzw. 10,3 cm Wuchshöhe auf und zeigte nach 30 Tagen ca. 80% Pilzbefall.

Mit handelsüblichem Propiconazol behandelte Weizensaat wies zum gleichen Zeitpunkt 1,4 cm bzw. 8,0 cm Wuchshöhe auf und zeigte ca. 40% Pilzbefall.

Dagegen wies die mit dem erfindungsgemässen 2R,4S-Isomeren behandelte Weizensaat zum gleichen Zeitpunkt eine Wuchshöhe von 2,1 cm bzw. 10,3 cm auf und zeigte einen Pilzbefall von unter 10%.

Gleichermassen wies die mit dem erfindungsgemässen 2RS,4S-Diasteromerengemisch des Propiconazols behandelte Weizensaat zum gleichen Zeitpunkt eine Wuchshöhe von 1,8 cm bzw. 10,1 cm auf und zeigte einen Pilzbefall von 10-15%.

### Beispiel 3.2: Beizwirkung gegen Fusarium nivale an Roggen

Natürlich mit Fusarium nivale infizierter Roggen wird auf einer Mischrolle mit der Prüfsubstanz gebeizt, wobei folgende Konzentrationen zur Anwendung gelangen: 50 ppm AS (= 5 g AS/100 kg Saatgut) und in einigen Fällen 100 ppm AS. Je 100 Samenkörner des infizierten und behandelten Roggens werden im Klimaraum in 40 $cm^2$ grosse Saatschalen eingesät und unter Stressbedingungen gehalten, die denen der natürlichen Wintersaat bei niederen Temperaturen und der Dunkelheit einer Schneedecke entsprechen. Während 4 Wochen werden die Saatschalen bei völliger Dunkelheit und bei +4°C gehalten, danach wird die Temperatur langsam auf 10°C erhöht und mit künstlicher Beleuchtung ein Tag- und Nacht-Wechsel simuliert.

Zur Ermittlung der Wirkstoffaktivität wird nach insgesamt 10 Wochen der prozentuale Anteil mit Fusarium befallener Pflanzen ausgezählt.

| | Zahl aufgelaufener Pflanzen | | davon befallene Pflanzen | |
|---|---|---|---|---|
| | bei 50 ppm AS | 100 ppm AS | bei 50 ppm AS | 100 ppm AS |
| 2R,4S-Isomeres | 89 | 91 | 6 | 3 |
| 2RS,4S-Diastereomeres | 89 | - | 9 | - |
| racem. Propiconazol | 80 | - | 12 | - |
| unbehandelte, aber infizierte Kontrolle | 60 | | 23 | |
| — = nicht geprüft | | | | |

### Beispiel 3.3: Beizwirkung gegen Helminthosporium gramineum an Gerste

Natürlich mit Helminthosporium gramineum infizierte Wintergerste der Sorte "CI" wird auf einer Mischrolle mit der Prüfsubstanz bei 25 ppm AS (= 2,5 g AS/100 kg Gerste) gebeizt.

[1] "Propiconazol" gemäss Pesticide Manual 8$^{th}$ Edition, S. 10170

Die infizierte und behandelte Gerste wird im Oktober im Freiland mit einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil mit Helminthosporium befallener Halme ausgezählt.

| | Befall | Pflanzenwuchs | |
|---|---|---|---|
| 2R,4S-Isomeres (99%-ig) | 0 % | normal | 90 % der Saat aufgelaufen |
| 2R,4S-Isomeres (70%-ig)* | 0 % | normal | |
| 2RS,4S-Diastereom. | 4 % | kaum beeinträchtigt | |
| racem. Propiconazol | 11 % | ca. 85 % der Saat aufgelaufen | |

* (hergestellt durch Vermischen von 20 Teilen 2R,4S und 30 Teilen 2RS,4S)

Unbehandelte jedoch infizierte Kontrollpflanzen wiesen einen Krankheitsbefall von 100 % auf.

<u>Beispiel 3.4</u>: <u>Beizwirkung gegen Ustilago tritici an Weizen</u>

Natürlich mit Ustilago nuda infizierter Winterweizen wird auf einer Mischrolle mit der Prüfsubstanz 25 ppm AS (= 2,5 g AS/100 kg Weizen) gebeizt.

Der infizierte und behandelte Weizen wird im Oktober im Freiland mit einer Sämaschine auf Parzellen von 2 m Länge und 3 Saatreihen ausgesät, mit 3 Wiederholungen pro Versuchsprodukt.

Bis zur Befallauswertung wird die Versuchspflanzung unter normalen Feldbedingungen kultiviert.

Zur Ermittlung der Wirkstoffaktivität wird während der Blüte der prozentuale Anteil vom Pilz befallener Aehren ausgezählt.

| | Befall | Pflanzenwuchs |
|---|---|---|
| 2R,4S-Isomeres | 0 % | normal |
| 2RS,4S-Diastereom. | 22 % | kaum beeinträchtigt (ca. 95 % der Saat aufgelaufen) |
| racem. Propiconazol | 43 % | ca. 80 % der Saat aufgelaufen |

Die Ergebnisse der vorstehenden Versuche 3.1 bis 3.4 belegen, dass handelsübliches Propiconazol wegen seiner Phytotoxizität für Saatgutbehandlung ungeeignet ist, hingegen das 2RS,4S-Diastereomerengemisch eine befriedigende und das 2R,4S-Isomere eine ungewöhnlich hohe Schutzwirkung erzielen, wobei letzteres die Keimfähigkeit von Getreidesaatgut nicht im geringsten beeinträchtigt.

<u>Beispiel 3.5</u> <u>Residual-protektive Wirkung gegen Venturia inaequalis auf Apfeltrieben</u>

Apfelstecklinge mit 10-20 cm langen Frischtrieben werden zum vergleichenden Demonstration unterschiedlicher Wuchshemmung mit einer aus Spritzpulver des Wirkstoffes hergestellten sehr verdünnten Spritzbrühe (50 ppm Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24 °C aufgestellt. Der Schorfbefall wird 15 Tage nach der Infektion beurteilt.

| | Befall | Wuchshemmung (Wuchs unbeeinflusst = 100 %) |
|---|---|---|
| 2R,4S-Isomeres (99%-ig) | 52 % | 97 % |
| 2R,4S-Isomeres (70%-ig)* | 58 % | 93 % |
| racem. Propiconazol | 77 % | 80 % |

* (hergestellt durch Vermischen von 20 Teilen 2R,4S und 30 Teilen 2RS,4S)

Die Einsatzmöglichkeit des 2R,4S-Isomeren und des 2RS,4S-Diastereomeren im Obstbau ist damit gegeben.

**Patentansprüche**

**Patentansprüche für folgende Vertragsstaaten : AT, BE, CH, DE, FR, GB, GR, IT, LI, LU, NL, SE**

1. Verfahren zur Herstellung des 2RS,4S-Diastereomerengemisches der Formel Ia und des 2R,4S-Isomeren der in Anspruch 10 gezeigten Formel I von 1-[2-(2,4-Dichlorphenyl)-4-n-propyl-1,3-dioxolan-2-ylmethyl]-1H-1,2,4-triazol, dadurch gekennzeichnet, dass man 2',4'-Dichlor-2-bromacetophenon mit S-1,2-Pentandiol zum Diastereomerengemisch 2RS,4S-2-[2-(2,4-Dichlorphenyl)-2-brommethyl-4-n-propyl-1,3-dioxolan der Formel III kondensiert und dieses mit 1,2,4-Triazol der Formel IV

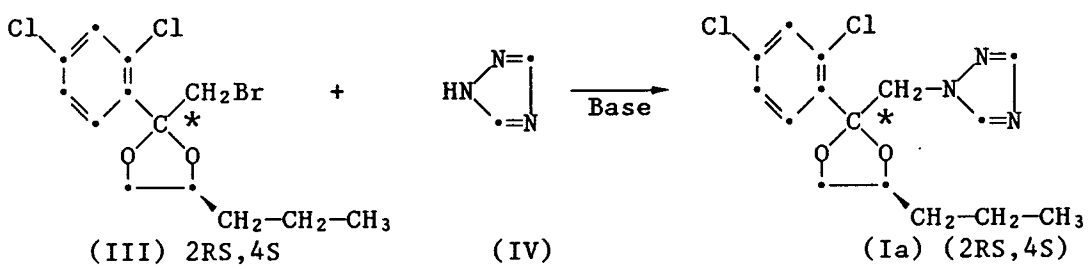


(III) 2RS,4S (IV) (Ia) (2RS,4S)

in einem Lösungsmittel bei 10-160 °C, in Gegenwart einer Base als Säureakzeptor, zum Diastereomerengemisch 2RS,4S-1-[2-(2,4-Dichlorphenyl)-4-n-propyl-1,3-dioxolan-2-ylmethyl]-1H-1,2,4-triazol der Formel Ia umsetzt und dieses isoliert und gewünschtenfalls aus dem erhaltenen 2RS,4S-Diastereomerengemisch das 2R,4S-1-[2-(2,4-Dichlorphenyl)-4-n-propyl-1,3-dioxolan-2-ylmethyl]-1H-1,2,4-triazol der Formel I abtrennt und isoliert.

2. Verfahren nach Anspruch 1, dadurch gekennzeichnet, dass man die Umsetzung der Verbindungen III und IV in einem apolaren-aprotischen Lösungsmittel in Gegenwart eines Alkalihydroxids durchführt.

3. Verfahren nach Anspruch 2, dadurch gekennzeichnet, dass man die Umsetzung in Dimethylsulfoxid bei 130-150 °C durchführt.

4. Verfahren nach Anspruch 2, dadurch gekennzeichnet, dass man die Umsetzung in Gegenwart von Kaliumhydroxid durchführt.

5. Verfahren nach Anspruch 1, dadurch gekennzeichnet, dass man die Abtrennung des 2R,4S-Diastereomeren an Kieselgel unter Verwendung von aliphatischen Kohlenwasserstoffen oder aliphatischen Estern oder deren Gemischen als Laufmittel durchführt.

6. Das Diastereomerengemisch 2RS,4S der Formel Ia von 1-[2-(2,4-Dichlorphenyl)-4-n-propyl-1,3-dioxolan-2-ylmethyl]-1H-1,2,4-triazol,

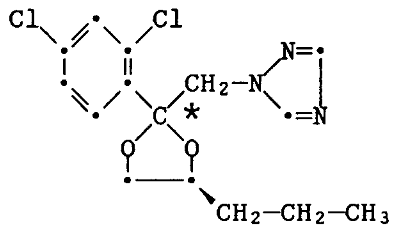

(Ia)
2RS,4S

in freier Form oder Salzform.

7. Mikrobizides Mittel enthaltend als aktive Komponente das 1-[2-(2,4-Dichlorphenyl)-4-n-propyl-1,3-dioxolan-2-ylmethyl]-1H-1,2,4-triazol ( = Propiconazol), dadurch gekennzeichnet, dass die aktive Komponente zu mindestens 90 Gew.-% aus dem 2RS,4S-Diastereomerengemisch der Formel Ia besteht.

8. Mittel gemäss Anspruch 7, dadurch gekennzeichnet, dass der Rest-Anteil der aktiven Komponente an 4R-Isomeren unter 5 Gew.-% liegt.

9. Mittel gemäss Anspruch 7, dadurch gekennzeichnet, dass die aktive Komponente frei von 4R-Isomeren ist.

10. Mikrobizides Mittel, enthaltend als aktive Komponente das 1-[2-(2,4-Dichlorphenyl)-4-n-propyl-1,3-dioxolan-2-ylmethyl]-1H-1,2,4-triazol, dadurch gekennzeichnet, dass die aktive Komponente zu mindestens 45 Gew.-% aus dem 2R,4S-Isomeren der Formel I

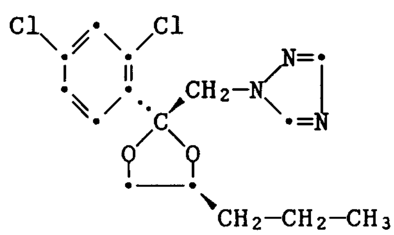

(I)
2R,4S

besteht.

11. Mittel gemäss Anspruch 10, dadurch gekennzeichnet, dass das 1-[2-(2,4-Dichlorphenyl)-4-n-propyl-1,3-dioxolan-2-ylmethyl]-1H-1,2,4-triazol zu mindestens 70 Gew.-% aus dem 2R,4S-Isomeren der Formel I besteht.

12. Mittel gemäss Anspruch 10, dadurch gekennzeichnet, dass das 1-[2-(2,4-Dichlorphenyl)-4-n-propyl-1,3-dioxolan-2-ylmethyl]-1H-1,2,4-triazol zu mindestens 90 Gew.-% aus dem 2R,4S-Isomeren der Formel I besteht.

13. Mittel gemäss Anspruch 10, dadurch gekennzeichnet, dass die aktive Komponente zu mindestens 98 Gew.-% aus dem 2R,4S-Isomeren der Formel I besteht.

14. Verfahren zur Bekämpfung oder Verhütung eines pathogenen Mikroorganismen-Befalls an Kulturpflanzen, dadurch gekennzeichnet, dass Propiconazol mit einem Gehalt von mindestens 90 Gew.-% an Diastereomerengemisch der Formel Ia auf die Pflanzen, auf Pflanzenteile oder auf den Standort der Pflanzen appliziert wird.

15. Verfahren gemäss Anspruch 14, dadurch gekennzeichnet, dass der Rest-Anteil an 4R-Isomeren im eingesetzten Propiconazol unter 5 Gew.-%, bezogen auf die Gesamtmenge an Propiconazol, liegt.

**16.** Verfahren gemäss Anspruch 14, dadurch gekennzeichnet, dass das eingesetzte Propiconazol frei von 4R-Isomeren ist.

**17.** Verfahren zur Bekämpfung oder Verhütung eines pathogenen Mikroorganismen-Befalls an Kulturpflanzen, dadurch gekennzeichnet, dass Propiconazol mit einem Gehalt von mindestens 45 Gew.-% an 2R,4S-Isomeren der Formel I auf die Pflanzen, auf Pflanzenteile oder auf den Standort der Pflanzen appliziert wird.

**18.** Verfahren gemäss Anspruch 17, dadurch gekennzeichnet, dass das eingesetzte Propiconazol zu mindestens 70 Gew.-% aus dem 2R,4S-Isomeren der Formel I besteht.

**19.** Verfahren gemäss Anspruch 17, dadurch gekennzeichnet, dass das eingesetzte Propiconazol zu mindestens 90 Gew.-% aus dem 2R,4S-Isomeren der Formel I besteht.

**20.** Verfahren gemäss Anspruch 17, dadurch gekennzeichnet, dass das eingesetzte Propiconazol zu mindestens 98 Gew.-% aus dem 2R,4S-Isomeren der Formel I besteht.

**21.** Verfahren gemäss Anspruch 14 oder 17, dadurch gekennzeichnet, dass Blattapplikation an Obstbäumen durchgeführt wird.

**22.** Verfahren gemäss Anspruch 14 oder 17, dadurch gekennzeichnet, dass die behandelten Pflanzenteile das Saatgut sind.

**23.** Verfahren gemäss Anspruch 22, dadurch gekennzeichnet, dass GetreideSaatgut gebeizt wird.

**24.** Das gemäss dem Verfahren von Anspruch 22 behandelte Saatgut.

**25.** Verfahren gemäss einem der Ansprüche 17 bis 20, dadurch gekennzeichnet, dass das eingesetzte Propiconazol frei von 4R-Isomeren ist.

**26.** Mittel gemäss einem der Ansprüche 10 bis 13, dadurch gekennzeichnet, dass das eingesetzte Propiconazol frei von 4R-Isomeren ist.

**Patentansprüche für folgenden Vertragsstaat : ES**

**1.** Verfahren zur Herstellung des 2RS,4S-Diastereomerengemisches der Formel Ia und des 2R,4S-Isomeren der in Anspruch 9 gezeigten Formel I von 1-[2-(2,4-Dichlorphenyl)-4-n-propyl-1,3-dioxolan-2-ylmethyl]-1H-1,2,4-triazol, dadurch gekennzeichnet, dass man 2',4'-Dichlor-2-bromacetophenon mit S-1,2-Pentandiol zum Diastereomerengemisch 2RS,4S-2-[2-(2,4-Dichlorphenyl)-2-brommethyl-4-n-propyl-1,3-dioxolan der Formel III kondensiert und dieses mit 1,2,4-Triazol der Formel IV

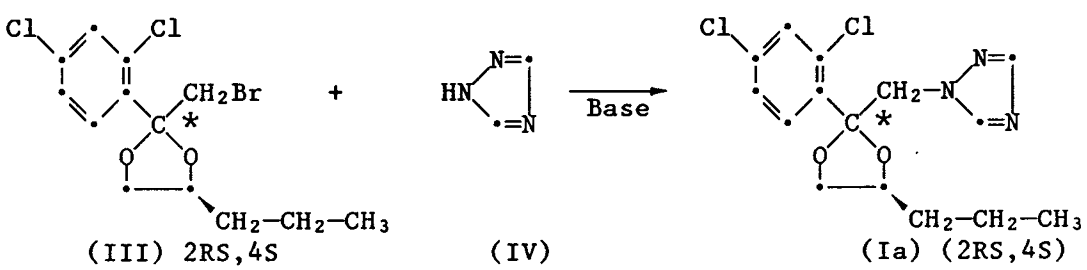


(III) 2RS,4S (IV) (Ia) (2RS,4S)

in einem Lösungsmittel bei 10-160 °C, in Gegenwart einer Base als Säureakzeptor, zum Diastereomerengemisch 2RS,4S-1-[2-(2,4-Dichlorphenyl)-4-n-propyl-1,3-dioxolan-2-ylmethyl]-1H-1,2,4-triazol der Formel Ia umsetzt und dieses isoliert und gewünschtenfalls aus dem erhaltenen 2RS,4S-Diastereomerengemisch das 2R,4S-1-[2-(2,4-Dichlorphenyl)-4-n-propyl-1,3-dioxolan-2-ylmethyl]-1H-1,2,4-triazol der Formel I abtrennt und isoliert.

**2.** Verfahren nach Anspruch 1, dadurch gekennzeichnet, dass man die Umsetzung der Verbindungen III und IV in einem apolaren-aprotischen Lösungsmittel in Gegenwart eines Alkalihydroxids durchführt.

**3.** Verfahren nach Anspruch 2, dadurch gekennzeichnet, dass man die Umsetzung in Dimethylsulfoxid bei 130-150 °C durchführt.

**4.** Verfahren nach Anspruch 2, dadurch gekennzeichnet, dass man die Umsetzung in Gegenwart von Kaliumhydroxid durchführt.

**5.** Verfahren nach Anspruch 1, dadurch gekennzeichnet, dass man die Abtrennung des 2R,4S-Diastereomeren an Kieselgel unter Verwendung von aliphatischen Kohlenwasserstoffen oder aliphatischen Estern oder deren Gemischen als Laufmittel durchführt.

**6.** Mikrobizides Mittel enthaltend als aktive Komponente das 1-[2-(2,4-Dichlorphenyl)-4-n-propyl-1,3-dioxolan-2-ylmethyl]-1H-1,2,4-triazol (= Propiconazol), dadurch gekennzeichnet, dass die aktive Komponente zu mindestens 90 Gew.-% aus dem 2RS,4S-Diastereomerengemisch der Formel Ia besteht.

**7.** Mittel gemäss Anspruch 6, dadurch gekennzeichnet, dass der Rest-Anteil der aktiven Komponente an 4R-Isomeren unter 5 Gew.-% liegt.

**8.** Mittel gemäss Anspruch 6, dadurch gekennzeichnet, dass die aktive Komponente frei von 4R-Isomeren ist.

**9.** Mikrobizides Mittel, enthaltend als aktive Komponente das 1-[2-(2,4-Dichlorphenyl)-4-n-propyl-1,3-dioxolan-2-ylmethyl]-1H-1,2,4-triazol, dadurch gekennzeichnet, dass die aktive Komponente zu mindestens 45 Gew.-% aus dem 2R,4S-Isomeren der Formel I

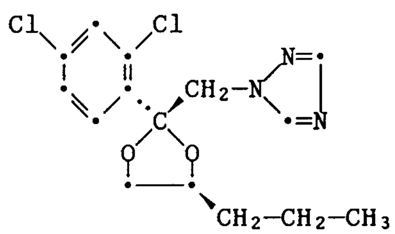

(I)
2R,4S

besteht.

**10.** Mittel gemäss Anspruch 9, dadurch gekennzeichnet, dass das 1-[2-(2,4-Dichlorphenyl)-4-n-propyl-1,3-dioxolan-2-ylmethyl]-1H-1,2,4-triazol zu mindestens 70 Gew.-% aus dem 2R,4S-Isomeren der Formel I besteht.

**11.** Mittel gemäss Anspruch 9, dadurch gekennzeichnet, dass das 1-[2-(2,4-Dichlorphenyl)-4-n-propyl-1,3-dioxolan-2-ylmethyl]-1H-1,2,4-triazol zu mindestens 90 Gew.-% aus dem 2R,4S-Isomeren der Formel I besteht.

**12.** Mittel gemäss Anspruch 9, dadurch gekennzeichnet, dass die aktive Komponente zu mindestens 98 Gew.-% aus dem 2R,4S-Isomeren der Formel I besteht.

**13.** Verfahren zur Bekämpfung oder Verhütung eines pathogenen Mikroorganismen-Befalls an Kulturpflanzen, dadurch gekennzeichnet, dass Propiconazol mit einem Gehalt von mindestens 90 Gew.-% an Diastereomerengemisch der Formel Ia auf die Pflanzen, auf Pflanzenteile oder auf den Standort der Pflanzen appliziert wird.

**14.** Verfahren gemäss Anspruch 14, dadurch gekennzeichnet, dass der Rest-Anteil an 4R-Isomeren im eingesetzten Propiconazol unter 5 Gew.-%, bezogen auf die Gesamtmenge an Propiconazol, liegt.

**15.** Verfahren gemäss Anspruch 14, dadurch gekennzeichnet, dass das eingesetzte Propiconazol frei von 4R-Isomeren ist.

**16.** Verfahren zur Bekämpfung oder Verhütung eines pathogenen Mikroorganismen-Befalls an Kulturpflanzen, dadurch gekennzeichnet, dass Propiconazol mit einem Gehalt von mindestens 45 Gew.-% an 2R,4S-Isomeren der Formel I auf die Pflanzen, auf Pflanzenteile oder auf den Standort der Pflanzen appliziert wird.

**17.** Verfahren gemäss Anspruch 16, dadurch gekennzeichnet, dass das eingesetzte Propiconazol zu mindestens 70 Gew.-% aus dem 2R,4S-Isomeren der Formel I besteht.

**18.** Verfahren gemäss Anspruch 16, dadurch gekennzeichnet, dass das eingesetzte Propiconazol zu mindestens 90 Gew.-% aus dem 2R,4S-Isomeren der Formel I besteht.

**19.** Verfahren gemäss Anspruch 16, dadurch gekennzeichnet, dass das eingesetzte Propiconazol zu mindestens 98 Gew.-% aus dem 2R,4S-Isomeren der Formel I besteht.

**20.** Verfahren gemäss Anspruch 13 oder 16, dadurch gekennzeichnet, dass Blattapplikation an Obstbäumen durchgeführt wird.

**21.** Verfahren gemäss Anspruch 13 oder 16, dadurch gekennzeichnet, dass die behandelten Pflanzenteile das Saatgut sind.

**22.** Verfahren gemäss Anspruch 21, dadurch gekennzeichnet, dass Getreide-Saatgut gebeizt wird.

**23.** Das gemäss dem Verfahren von Anspruch 21 behandelte Saatgut.

**24.** Verfahren gemäss einem der Ansprüche 16 bis 19, dadurch gekennzeichnet, dass das eingesetzte Propiconazol frei von 4R-Isomeren ist.

**25.** Mittel gemäss einem der Ansprüche 9 bis 12, dadurch gekennzeichnet, dass das eingesetzte Propiconazol frei von 4R-Isomeren ist.

**Claims**

**Claims for the following Contracting States : AT, BE, CH, DE, FR, GB, GR, IT, LI, LU, NL, SE**

**1.** A process for the preparation of the 2RS,4S-diastereoisomeric mixture of formula Ia and the 2R,4S-isomer of formula I, shown in claim 10, of 1-[2-(2,4-dichlorophenyl)-4-n-propyl-1,3-dioxolan-2-ylmethyl]-1H-1,2,4-triazole, which comprises condensing 2',4'-dichloro-2-bromoacetophenone with S-1,2-pentanediol to give the diastereoisomeric mixture 2RS,4S-2-[2-(2,4-dichlorophenyl)-2-bromomethyl-4-n-propyl-1,3-dioxolane of formula III, and reacting the latter with 1,2,4-triazole of formula IV

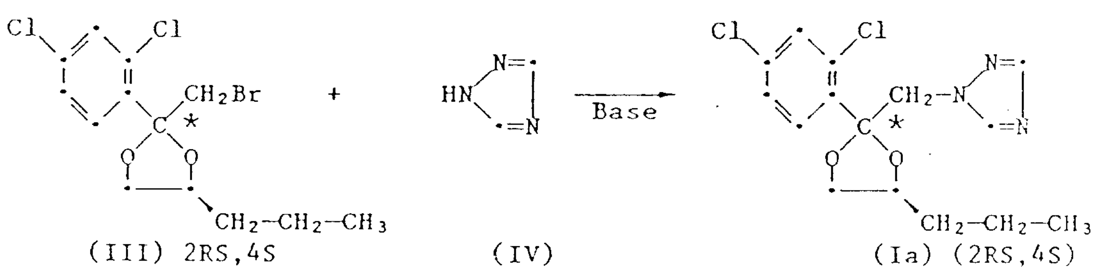


(III) 2RS,4S (IV) (Ia) (2RS,4S)

in a solvent at from 10 to 160 °C, in the presence of a base as acid acceptor, to give the diastereoisomeric mixture 2RS,4S-1-[2-(2,4-dichlorophenyl)-4-n-propyl-1,3-dioxolan-2-ylmethyl]-1H-1,2,4-triazole of formula Ia and isolating it and, if desired, separating 2R,4S-1-[2-(2,4-dichlorophenyl)-4-n-propyl-1,3-dioxolan-2-ylmethyl]-1H-1,2,4-triazole of formula I from the resulting 2RS,4S-diastereoisomeric mixture and isolating it.

2. A process according to claim 1 wherein the reaction of compounds III and IV is carried out in an apolar-aprotic solvent in the presence of an alkali metal hydroxide.

3. A process according to claim 2 wherein the reaction is carried out in dimethyl sulfoxide at from 130 to 150 °C.

4. A process according to claim 2 wherein the reaction is carried out in the presence of potassium hydroxide.

5. A process according to claim 1 wherein the 2R,4S-diastereoisomer is separated over silica gel using aliphatic hydrocarbons or aliphatic esters or mixtures thereof as eluants.

6. The diastereoisomeric mixture 2RS,4S of formula Ia of 1-[2-(2,4-dichlorophenyl)-4-n-propyl-1,3-dioxolan-2-ylmethyl]-1H-1,2,4-triazole

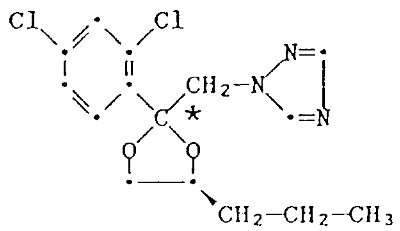

(Ia)
2RS,4S

in free form or in salt form.

7. A microbicidal composition comprising as active ingredient 1-[2-(2,4-dichlorophenyl)-4-n-propyl-1,3-dioxolan-2-ylmethyl]-1H-1,2,4-triazole (= propiconazole), wherein the 2RS,4S-diastereoisomeric mixture of formula Ia forms at least 90 % by weight of the active ingredient.

8. A composition according to claim 7 wherein the residual portion of the active ingredient of 4R-isomers is less than 5 % by weight.

9. A composition according to claim 7 wherein the active ingredient is free of 4R-isomers.

10. A microbicidal composition comprising as active ingredient 1-[2-(2,4-dichlorophenyl)-4-n-propyl-1,3-dioxolan-2-ylmethyl]-1H-1,2,4-triazole, wherein the 2R,4S-isomer of formula I

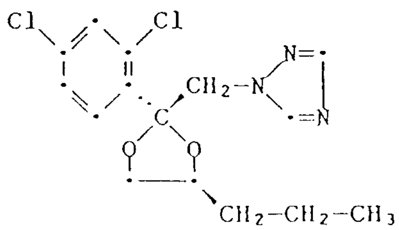

(I)
2R,4S

forms at least 45 % by weight of the active ingredient.

11. A composition according to claim 10 wherein the 2R,4S-isomer of formula I forms at least 70 % by weight of the 1-[2-(2,4-dichlorophenyl)-4-n-propyl-1,3-dioxolan-2-ylmethyl]-1H-1,2,4-triazole.

12. A composition according to claim 10 wherein the 2R,4S-isomer of formula I forms at least 90 % by weight of the 1-[2-(2,4-dichlorophenyl)-4-n-propyl-1,3-dioxolan-2-ylmethyl]-1H-1,2,4-triazole.

**13.** A composition according to claim 10 wherein the 2R,4S-isomer of formula I forms at least 98 % by weight of the active ingredient.

**14.** A method of controlling or preventing attack by pathogenic microorganisms on cultivated plants, which comprises applying propiconazole comprising at least 90 % by weight of the diastereoisomeric mixture of formula Ia to the plant, to parts of plants, or to the locus thereof.

**15.** A method according to claim 14 wherein the residual portion of 4R-isomers in the propiconazole used is less than 5 % by weight, based on the total amount of propiconazole.

**16.** A method according to claim 14 wherein the propiconazole used is free of 4R-isomers.

**17.** A method of controlling or preventing attack by pathogenic microorganisms on cultivated plants, which comprises applying propiconazole comprising at least 45 % by weight of the 2R,4S-isomer of formula I to the plant, to parts of plants, or to the locus thereof.

**18.** A method according to claim 17 wherein the 2R,4S-isomer of formula I forms at least 70 % by weight of the propiconazole used.

**19.** A method according to claim 17 wherein the 2R,4S-isomer of formula I forms at least 90 % by weight of the propiconazole used.

**20.** A method according to claim 17 wherein the 2R,4S-isomer of formula I forms at least 98 % by weight of the propiconazole used.

**21.** A method according to either claim 14 or claim 17 wherein foliar application is carried out on fruit trees.

**22.** A method according to either claim 14 or claim 17 wherein the parts of plants treated are the seeds.

**23.** A method according to claim 22 wherein cereal seeds are dressed.

**24.** Seeds treated according to the method of claim 22.

**25.** A method according to any one of claims 17 to 20 wherein the propiconazole used is free of 4R-isomers.

**26.** A composition according to any one of claims 10 to 13 wherein the propiconazole used is free of 4R-isomers.

**Claims for the following Contracting State : ES**

**1.** A process for the preparation of the 2RS,4S-diastereoisomeric mixture of formula Ia and the 2R,4S-isomer of formula I, shown in claim 9, of 1-[2-(2,4-dichlorophenyl)-4-n-propyl-1,3-dioxolan-2-ylmethyl]-1H-1,2,4-triazole, which comprises condensing 2',4'-dichloro-2-bromoacetophenone with S-1,2-pentanediol to give the diastereoisomeric mixture 2RS,4S-2-[2-(2,4-dichlorophenyl)-2-bromomethyl-4-n-propyl-1,3-dioxolane of formula III, and reacting the latter with 1,2,4-triazole of formula IV

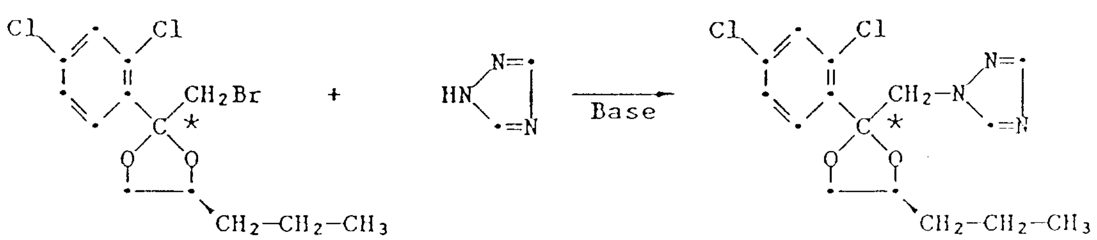


(III) 2RS,4S (IV) (Ia) (2RS,4S)

in a solvent at from 10 to 160 °C, in the presence of a base as acid acceptor, to give the diastereoisomeric mixture 2RS,4S-1-[2-(2,4-dichlorophenyl)-4-n-propyl-1,3-dioxolan-2-ylmethyl]-1H-1,2,4-triazole of formula Ia and isolating it and, if desired, separating 2R,4S-1-[2-(2,4-dichlorophenyl)-4-n-propyl-1,3-dioxolan-2-ylmethyl]-1H-1,2,4-triazole of formula I from the resulting 2RS,4S-diastereoisomeric mixture and isolating it.

**2.** A process according to claim 1 wherein the reaction of compounds III and IV is carried out in an apolar-aprotic solvent in the presence of an alkali metal hydroxide.

**3.** A process according to claim 2 wherein the reaction is carried out in dimethyl sulfoxide at from 130 to 150 °C.

**4.** A process according to claim 2 wherein the reaction is carried out in the presence of potassium hydroxide.

**5.** A process according to claim 1 wherein the 2R,4S-diastereoisomer is separated over silica gel using aliphatic hydrocarbons or aliphatic esters or mixtures thereof as eluants.

**6.** A microbicidal composition comprising as active ingredient 1-[2-(2,4-dichlorophenyl)-4-n-propyl-1,3-dioxolan-2-ylmethyl]-1H-1,2,4-triazole (= propiconazole), wherein the 2RS,4S-diastereoisomeric mixture of formula Ia forms at least 90 % by weight of the active ingredient.

**7.** A composition according to claim 6 wherein the residual portion of the active ingredient of 4R-isomers is less than 5 % by weight.

**8.** A composition according to claim 6 wherein the active ingredient is free of 4R-isomers.

**9.** A microbicidal composition comprising as active ingredient 1-[2-(2,4-dichlorophenyl)-4-n-propyl-1,3-dioxolan-2-ylmethyl]-1H-1,2,4-triazole, wherein the 2R,4S-isomer of formula I

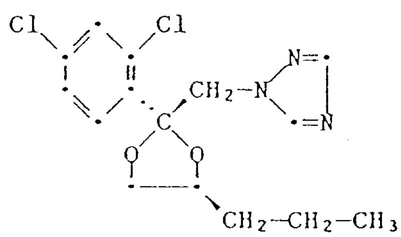

(I)
2R,4S

forms at least 45 % by weight of the active ingredient.

**10.** A composition according to claim 9 wherein the 2R,4S-isomer of formula I forms at least 70 % by weight of the 1-[2-(2,4-dichlorophenyl)-4-n-propyl-1,3-dioxolan-2-ylmethyl]-1H-1,2,4-triazole.

**11.** A composition according to claim 9 wherein the 2R,4S-isomer of formula I forms at least 90 % by weight of the 1-[2-(2,4-dichlorophenyl)-4-n-propyl-1,3-dioxolan-2-ylmethyl]-1H-1,2,4-triazole.

**12.** A composition according to claim 9 wherein the 2R,4S-isomer of formula I forms at least 98 % by weight of the active ingredient.

**13.** A method of controlling or preventing attack by pathogenic microorganisms on cultivated plants, which comprises applying propiconazole comprising at least 90 % by weight of the diastereoisomeric mixture of formula Ia to the plant, to parts of plants, or to the locus thereof.

**14.** A method according to claim 13 wherein the residual portion of 4R-isomers in the propiconazole used is less than 5 % by weight, based on the total amount of propiconazole.

**15.** A method according to claim 14 wherein the propiconazole used is free of 4R-isomers.

**16.** A method of controlling or preventing attack by pathogenic microorganisms on cultivated plants, which comprises applying propiconazole comprising at least 45 % by weight of the 2R,4S-isomer of formula I to the plant, to parts of plants, or to the locus thereof.

**17.** A method according to claim 16 wherein the 2R,4S-isomer of formula I forms at least 70 % by weight of the propiconazole used.

**18.** A method according to claim 16 wherein the 2R,4S-isomer of formula I forms at least 90 % by weight of the propiconazole used.

**19.** A method according to claim 16 wherein the 2R,4S-isomer of formula I forms at least 98 % by weight of the propiconazole used.

**20.** A method according to either claim 13 or claim 16 wherein foliar application is carried out on fruit trees.

**21.** A method according to either claim 13 or claim 16 wherein the parts of plants treated are the seeds.

**22.** A method according to claim 21 wherein cereal seeds are dressed.

**23.** Seeds treated according to the method of claim 21.

**24.** A method according to any one of claims 16 to 19 wherein the propiconazole used is free of 4R-isomers.

**25.** A composition according to any one of claims 9 to 12 wherein the propiconazole used is free of 4R-isomers.

**Revendications**
**Revendications pour les Etats contractants suivants : AT, BE, CH, DE, FR, GB, GR, IT, LI, LU, NL, SE**

**1.** Procédé de préparation du mélange de diastéréoisomères 2RS,4S de formule Ia et de l'isomère 2R,4S, répondant à la formule I de la revendication 10, du 1-[2-(2,4-dichlorophényl)-4-n-propyl-1,3-dioxoianne-2-ylméthyl]-1H-1,2,4-triazole, caractérisé en ce que l'on condense la 2',4'-dichloro-2-bromo-acétophénone avec le S-1,2-pentane-diol, ce qui donne le mélange de diastéréoisomères 2RS,4S-2-[2-(2,4-dichlorophényl)-2-bromométhyl-4-n-propyl-1,3-dioxolanne de formule III qu'on fait réagir avec le 1,2,4-triazole de formule IV

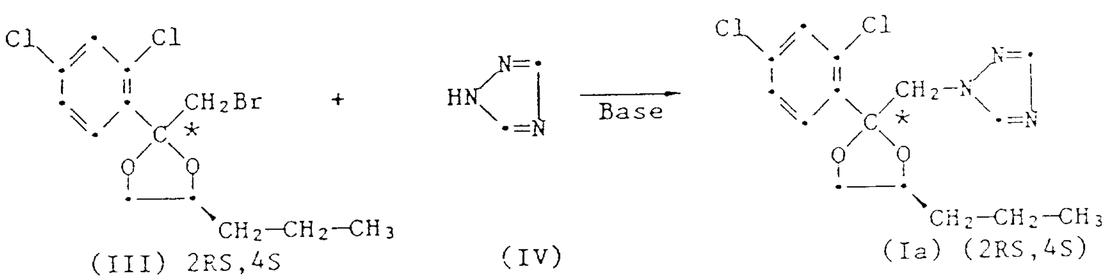

(III) 2RS,4S (IV) (Ia) (2RS,4S)

dans un solvant à une température de 10 à 160 °C en présence d'une base qui sert d'accepteur d'acides, la réaction donnant le mélange de diastéréoisomères 2RS,4S-1-[2-(2,4-dichlorophényl)-4-n-propyl-1,3-dioxolanne-2-ylméthyl]-1H-1,2,4-triazole de formule Ia qu'on isole et d'où, si on le désire, on sépare et on isole le 2R,4S-1-[2-(2,4-dichlorophényl)-4-n-propyl-1,3-dioxolanne-2-ylméthyl]-1H-1,2,4-triazole de formule I.

**2.** Procédé selon revendication 1, caractérisé en ce que la réaction entre les composés III et IV est effectuée dans un solvant polaire aprotonique en présence d'un hydroxyde alcalin.

3. Procédé selon revendication 2, caractérisé en ce que la réaction est effectuée dans le diméthylsulfoxyde à des températures de 130 à 150 °C.

4. Procédé selon revendication 2, caractérisé en ce que la réaction est effectuée en présence d'hydroxyde de potassium.

5. Procédé selon revendication 1, caractérisé en ce que la séparation du diastéréoisomère 2R,4S est effectuée sur gel de silice avec utilisation d'hydrocarbures aliphatiques ou d'esters aliphatiques ou leurs mélanges en tant qu'éluants.

6. Le mélange de diastéréoisomères 2RS,4S de formule Ia du 1-[2-(2,4-dichlorophényl)-4-n-propyl-1,3-dioxolanne-2-ylméthyl]-1H-1,2,4-triazole

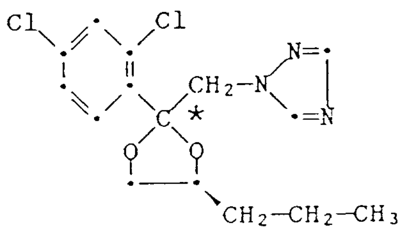

(Ia)
2RS,4S

à l'état libre ou à l'état de sel.

7. Produit microbicide contenant en tant que composant actif le 1-[2-(2,4-dichlorophényl)-4-n-propyl-1,3-dioxolanne-2-ylméthyl]-1H-1,2,4-triazole (= Propiconazol), caractérisé en ce que le composant actif consiste pour au moins 90 % en poids en le mélange de diastéréoisomères 2RS,4S de formule Ia.

8. Produit selon revendication 7, caractérisé en ce que la teneur résiduelle du composant actif en l'isomère 4R est inférieure à 5 % en poids.

9. Produit selon revendication 7, caractérisé en ce que le composant actif est exempt d'isomère 4R.

10. Produit microbicide contenant en tant que composant actif le 1-[2-(2,4-dichlorophényl)-4-n-propyl-1,3-dioxolanne-2-ylméthyl]-1H-1,2,4-triazole, caractérisé en ce que le composant actif consiste pour au moins 45 % en poids en l'isomère 2R,4S de formule I

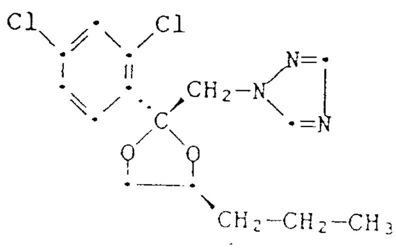

(I)
2R,4S

11. Produit selon revendication 10, caractérisé en ce que le 1-[2-(2,4-dichlorophényl)-4-n-propyl-1,3-dioxolanne-2-ylméthyl]-1H-1,2,4-triazole consiste pour au moins 70 % en poids en l'isomère 2R,4S de formule I.

12. Produit selon revendication 10, caractérisé en ce que le 1-[2-(2,4-dichlorophényl)-4-n-propyl-1,3-dioxolanne-2-ylméthyl]-1H-1,2,4-triazole consiste pour au moins 90 % en poids en l'isomère 2R,4S de formule I.

13. Produit selon revendication 10, caractérisé en ce que le composant actif consiste pour au moins 98 % en poids en l'isomère 2R,4S de formule I.

**14.** Procédé pour combattre ou prévenir une infection de microorganismes pathogènes sur des végétaux cultivés, caractérisé en ce que l'on applique sur les végétaux, des parties des végétaux ou l'habitat des végétaux, du Propiconazol à une teneur d'au moins 90 % en poids en le mélange de diastéréoisomères de formule Ia.

**15.** Procédé selon revendication 14, caractérisé en ce que la teneur résiduelle en isomère 4R du Propiconazol utilisé est inférieure à 5 % du poids total du Propiconazol.

**16.** Procédé selon revendication 14, caractérisé en ce que le Propiconazol utilisé est exempt de l'isomère 4R.

**17.** Procédé pour combattre ou prévenir une infection par microorganismes pathogènes de végétaux cultivés, caractérisé en ce que l'on applique sur les végétaux, des parties des végétaux ou l'habitat des végétaux, du Propiconazol à une teneur d'au moins 45 % en poids en l'isomère 2R,4S de formule I.

**18.** Procédé selon revendication 17, caractérisé en ce que le Propiconazol utilisé consiste pour au moins 70 % en poids en l'isomère 2R,4S de formule I.

**19.** Procédé selon revendication 17, caractérisé en ce que le Propiconazol utilisé consiste pour au moins 90 % en poids en l'isomère 2R,4S de formule I.

**20.** Procédé selon revendication 17, caractérisé en ce que le Propiconazol utilisé consiste pour au moins 98 % en poids en l'isomère 2R,4S de formule I.

**21.** Procédé selon revendication 14 ou 17, caractérisé en ce que l'application est faite sur le feuillage d'arbres fruitiers.

**22.** Procédé selon revendication 14 ou 17, caractérisé en ce que les parties de végétaux traitées sont les semences.

**23.** Procédé selon revendication 22, caractérisé en ce que l'on désinfecte des semences de céréales.

**24.** Les semences traitées par le procédé de la revendication 22.

**25.** Procédé selon une des revendications 17 à 20, caractérisé en ce que le Propiconazol utilisé est exempt de l'isomère 4R.

**26.** Produit selon une des revendications 10 à 13, caractérisé en ce que le Propiconazol utilisé est exempt de l'isomère 4R.

**Revendications pour l'Etat contractant suivant : ES**

**1.** Procédé de préparation du mélange de diastéréoisomères 2RS,4S de formule Ia et de l'isomère 2R,4S, répondant à la formule I de la revendication 9, du 1-[2-(2,4-dichlorophényl)-4-n-propyl-1,3-dioxolanne-2-ylméthyl]-1H-1,2,4-triazole, caractérisé en ce que l'on condense la 2',4'-dichloro-2-bromo-acétophénone avec le S-1,2-pentane-diol, ce qui donne le mélange de diastéréoisomères 2RS,4S-2-[2-(2,4-dichlorophényl)-2-bromométhyl-4-n-propyl-1,3-dioxolanne de formule III qu'on fait réagir avec le 1,2,4-triazole de formule IV

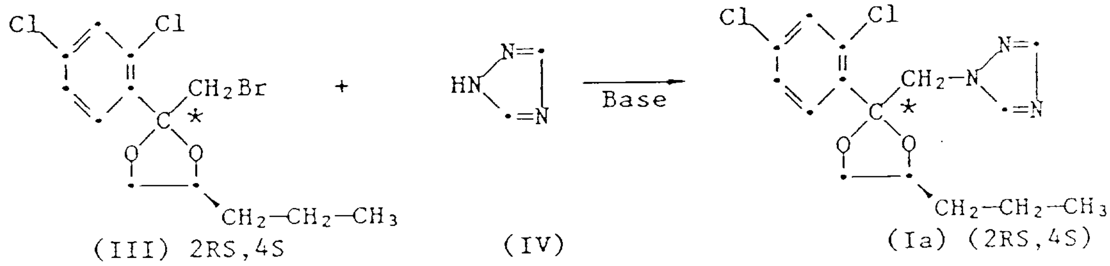


(III) 2RS,4S + (IV) (Ia) (2RS,4S)

dans un solvant à une température de 10 à 160 °C en présence d'une base qui sert d'accepteur d'acides, la réaction donnant le mélange de diastéréoisomères 2RS,4S-1-[2-(2,4-dichlorophényl)-4-n-propyl-1,3-dioxolanne-2-ylméthyl]-1H-1,2,4-triazole de formule Ia qu'on isole et d'où, si on le désire, on sépare et on isole le 2R,4S-1-[2-(2,4-dichlorophényl)-4-n-propyl-1,3-dioxolanne-2-ylméthyl]-1H-1,2,4-triazole de formule I.

**2.** Procédé selon revendication 1, caractérisé en ce que la réaction entre les composés III et IV est effectuée dans un solvant polaire aprotonique en présence d'un hydroxyde alcalin.

**3.** Procédé selon revendication 2, caractérisé en ce que la réaction est effectuée dans le diméthyl-sulfoxyde à des températures de 130 à 150 °C.

**4.** Procédé selon revendication 2, caractérisé en ce que la réaction est effectuée en présence d'hydroxyde de potassium.

**5.** Procédé selon revendication 1, caractérisé en ce que la séparation du diastéréoisomère 2R,4S est effectuée sur gel de silice avec utilisation d'hydrocarbures aliphatiques ou d'esters aliphatiques ou leurs mélanges en tant qu'éluants.

**6.** Produit microbicide contenant en tant que composant actif le 1-[2-(2,4-dichlorophényl)-4-n-propyl-1,3-dioxolanne-2-ylméthyl]-1H-1,2,4-triazole (= Propiconazol), caractérisé en ce que le composant actif consiste pour au moins 90 % en poids en le mélange de diastéréoisomères 2RS,4S de formule Ia.

**7.** Produit selon revendication 6, caractérisé en ce que la teneur résiduelle du composant actif en l'isomère 4R est inférieure à 5 % en poids.

**8.** Produit selon revendication 6, caractérisé en ce que le composant actif est exempt d'isomère 4R.

**9.** Produit microbicide contenant en tant que composant actif le 1-[2-(2,4-dichlorophényl)-4-n-propyl-1,3-dioxolanne-2-ylméthyl]-1H-1,2,4-triazole, caractérisé en ce que le composant actif consiste pour au moins 45 % en poids en l'isomère 2R,4S de formule I

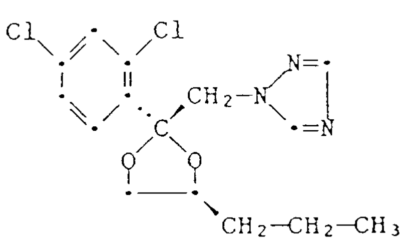

(I)
2R,4S

**10.** Produit selon revendication 9, caractérisé en ce que le 1-[2-(2,4-dichlorophényl)-4-n-propyl-1,3-dioxolanne-2-ylméthyl]-1H-1,2,4-triazole consiste pour au moins 70 % en poids en l'isomère 2R,4S de formule I.

**11.** Produit selon revendication 9, caractérisé en ce que le 1-[2-(2,4-dichlorophényl)-4-n-propyl-1,3-dioxolanne-2-ylméthyl]-1H-1,2,4-triazole consiste pour au moins 90 % en poids en l'isomère 2R,4S de formule I.

**12.** Produit selon revendication 9, caractérisé en ce que le composant actif consiste pour au moins 98 % en poids en l'isomère 2R,4S de formule I.

**13.** Procédé pour combattre ou prévenir l'infection de végétaux cultivés par des microorganismes pathogènes, caractérisé en ce que l'on applique sur les végétaux, des parties des végétaux ou l'habitat des végétaux, du Propiconazol à une teneur d'au moins 90 % en poids en le mélange de diastéréoisomères de formule Ia.

**14.** Procédé selon revendication 13, caractérisé en ce que la teneur résiduelle du Propiconazol utilisé en l'isomère 4R est inférieure à 5 % du poids total du Propiconazol.

**15.** Procédé selon revendication 14, caractérisé en ce que le Propiconazol utilisé est exempt de l'isomère 4R.

**16.** Procédé pour combattre ou prévenir une infection de végétaux cultivés par des microorganismes pathogènes, caractérisé en ce que l'on applique sur les végétaux, des parties des végétaux ou l'habitat des végétaux, du Propiconazol à une teneur d'au moins 45 % en poids en l'isomère 2R,4S de formule I.

**17.** Procédé selon revendication 16, caractérisé en ce que le Propiconazol utilisé consiste pour au moins 70 % en poids en l'isomère 2R,4S de formule I.

**18.** Procédé selon revendication 16, caractérisé en ce que le Propiconazol utilisé consiste pour au moins 90 % en poids en l'isomère 2R,4S de formule I.

**19.** Procédé selon revendication 16, caractérisé en ce que le Propiconazol utilisé consiste pour au moins 98 % en poids en l'isomère 2R,4S de formule I.

**20.** Procédé selon revendication 13 ou 16, caractérisé en ce que l'application est faite sur le feuillage d'arbres fruitiers.

**21.** Procédé selon revendication 13 ou 16, caractérisé en ce que les parties de végétaux traitées sont des semences.

**22.** Procédé selon revendication 21, caractérisé en ce que l'on désinfecte des semences de céréales.

**23.** Les semences traitées par le procédé de la revendication 21.

**24.** Procédé selon une des revendications 16 à 19, caractérisé en ce que le Propiconazol utilisé est exempt de l'isomère 4R.

**25.** Produit selon une des revendications 9 à 12, caractérisé en ce que le Propiconazol utilisé est exempt de l'isomère 4R.